Europäisches Patentamt

(19) European Patent Office

Office européen des brevets

(11) EP 0 550 342 B1

## (12) FASCICULE DE BREVET EUROPEEN

(45) Date de publication et mention
de la délivrance du brevet:
**18.06.1997 Bulletin 1997/25**

(51) Int. Cl.⁶: **A61N 1/368**

(21) Numéro de dépôt: **92403578.5**

(22) Date de dépôt: **30.12.1992**

(54) **Commande d'un stimulateur cardiaque en cas d'extra-systole ventriculaire**

Steuerung eines Herzschrittmachers bei Extra-Systolen

Controlling a pacemaker in case of extra-systoles

(84) Etats contractants désignés:
**BE CH DE ES FR GB IT LI SE**

(30) Priorité: **31.12.1991 FR 9116366**

(43) Date de publication de la demande:
**07.07.1993 Bulletin 1993/27**

(73) Titulaire: **ELA MEDICAL (Société anonyme)**
**F-92541 Montrouge Cédex (FR)**

(72) Inventeurs:
• **Nitzsche, Rémi**
  **F-78650 Beynes (FR)**

• **Limousin, Marcel**
  **F-92120 Montrouge (FR)**
• **Rosset, Nicolas**
  **F-92120 Montrouge (FR)**

(74) Mandataire: **Laget, Jean-Loup**
**Cabinet Loyer,**
**78, avenue Raymond Poincaré**
**75116 Paris (FR)**

(56) Documents cités:
EP-A- 0 241 102      EP-A- 0 360 668
EP-A- 0 436 517      WO-A-83/01389
DD-A-  221 372       FR-A- 2 544 989

Printed by Rank Xerox (UK) Business Services
2.14.7/3.4

## Description

L'invention concerne un stimulateur cardiaque mettant en oeuvre un procédé de commande du stimulateur cardiaque en cas d'extra-systole ventriculaire ou ESV.

Le document DD 221 372 décrit un procédé de stimulation synchrone de l'oreillette lors de la détection d'une ESV.

Le document EP A-0 436 517 décrit une méthode qui, sur la détection d'une tachycardie, fait varier les délais entre stimulation/détection auriculaire et venrriculaire.

Le document FR-A-2.544.989 décrit une méthode de lissage de la fréquence de la stimulation ventriculaire en cas de tachycardie auriculaire.

Le but principal de l'invention est d'éviter la pause compensatrice après une ESV, cette pause pouvant induire une tachycardie ventriculaire chez certains patients.

Un autre but de l'invention est de proposer un stimulateur cardiaque mettant en oeuvre un procédé de commande du stimulateur cardiaque qui, après détection d'une ESV, permette de retrouver une conduction auriculoventriculaire spontanée, tout en assurant une fréquence ventriculaire élevée pour améliorer l'hémodynamique du patient.

En cas de détection d'une ESV, il est souhaitable d'éviter l'installation d'une tachycardie ré-entrante ou TRE. En effet, l'ESV peut induire une dépolarisation de l'oreillette qui peut, à son tour, induire une stimulation du ventricule, et ainsi de suite, et il peut en résulter une TRE.

Un but de l'invention est de proposer un stimulateur cardiaque mettant en oeuvre un procédé de commande du stimulateur cardiaque, qui tende à éviter l'installation d'une TRE après détection d'une ESV.

La présente invention a pour objet un stimulateur cardiaque dans lequel en cas de détection d'une d'extra-systole ventriculaire ESV, une stimulation synchrone de l'oreillette est déclenchée , caractérisé en ce que, sur la détection de l'extra-systole, un algorithme est mis en jeu pour déclencher en outre : une commande de la stimulation de l'oreillette à un rythme plus rapide que celui du ventricule pendant quelques cycles cardiaques, une commande de la stimulation synchrone du ventricule pendant une période multiple d'un nombre programmé de cycles cardiaques, et après ce nombre programmé de cycles cardiaques, une diminution du rythme de la commande de la stimulation du ventricule, jusqu'à atteindre la fréquence de base programmée ou retrouver une détection sinusale.

Selon d'autres caractéristiques de l'invention :

- l'ESV sert de point de départ à un intervalle d'échappement ventriculaire IEV et à un intervalle ventriculo-auriculaire VA plus court que l'IEV ;

- l'IEV est égal à un pourcentage de l'intervalle moyen entre deux ondes P successives, compris entre 50 et 100 et de préférence égal à 87,5% ;

- pendant le nombre programmé de cycles, de préférence égal à 20, l'intervalle d'échappement ventriculaire IEV est maintenu constant, et l'intervalle ventriculo-auriculaire VA est réduit, à chaque cycle, tandis que le délai auriculo-ventriculaire DAV est augmenté de la durée correspondante pour respecter l'égalité : IEV = VA + DAV

- après le nombre programmé de cycles, de préférence égal à 20, l'IEV est augmenté et reste constant pour ledit nombre programmé de cycles ;

- le nombre programmé de cycles est compté par un compteur initialisé par la détection de l'ESV ;

- l'algorithme n'est mis en jeu que si un pourcentage de l'intervalle moyen PPm entre deux ondes P successives est supérieur à une durée prédéterminée, de préférence égale à 500 ms ;

- en cas de détection d'une nouvelle ESV fréquente, le rythme ventriculaire est de nouveau augmenté d'une pente d'accélération ;

- si le rythme ventriculaire accéléré correspond à un intervalle d'échappement ventriculaire inférieur à 500 ms, alors l'algorithme est désactivé ;

- après désactivation de l'algorithme, l'intervalle d'échappement ventriculaire est allongé par paliers jusqu'à retrouver la période de base ou un rythme sinusal.

D'autres caractéristiques ressortent de la description qui suit faite avec référence au dessin annexé sur lequel on peut voir :

Fig. 1 : un schéma représentatif d'opérations déclenchées sur une ESV par le procédé selon l'invention.

Fig 2 : Une courbe représentative des variations de l'intervalle ventriculaire en cas de détection d'une ESV isolée.

Fig 3 : Une représentation de l'intervalle ventriculaire en cas d'ESV rapprochées, conduisant à l'état de désactivation de l'algorithme mis en jeu dans le procédé selon l'invention.

Fig 4 : un diagramme d'états représentatif du fonctionnement de l'algorithme.

Il est possible de définir deux types d'ESV. Une ESV de type 1 correspond à une détection ventriculaire non précédée d'un événement auriculaire dans un intervalle de temps compris entre 31 et 300 ms, par exemple.

Une ESV de type 2 correspond à une détection ventriculaire, précédée d'un événement auriculaire dans un intervalle de temps compris entre 31 et 300 ms, dans le cas où le délai auriculo-ventriculaire AR est inférieur de plus de 31 ms au délai auriculo-ventriculaire DAV du cycle cardiaque précédent : DAV - AR > 31ms .

Dans la suite de la description, les ESV sont indifféremment de type 1 ou de type 2.

Par ailleurs, les ESV sont classées en deux catégories : une ESV est dite fréquente, ou critique, lorsqu'elle est précédée par un nombre de cycles cardiaques synchrones inférieur à un nombre programmé, par exemple 10 ; une ESV est dite non fréquente dans le cas contraire. Dans la suite de la description, et notamment sur les dessins, la première ESV détectée est par hypothèse une ESV non fréquente, et les autres ESV sont considérées comme fréquentes.

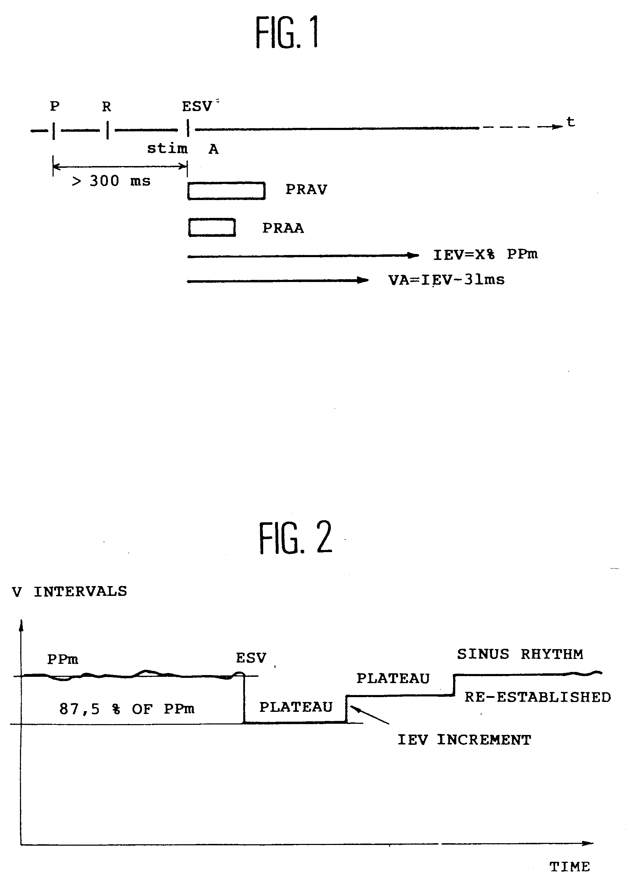

La figure 1 représente les différents délais ou intervalles qui sont déclenchés lors de la détection d'une ESV.

La figure 2 représente les variations de la fréquence cardiaque correspondant à l'application de l'algorithme mis en jeu dans le procédé mis en oeuvre dans un stimulateur selon l'invention. En cas de détection d'une ESV, non fréquente, la fréquence cardiaque est augmentée : l'intervalle d'échappement ventriculaire IEV est ramené à 87,5% par exemple de l'intervalle PPm, c'est-à-dire de l'intervalle moyen entre deux ondes P consécutives. Après un palier, l'IEV est allongé d'une pente "IEV increment" et maintenu constant pendant un deuxième palier, puis il est allongé de nouveau et maintenu constant jusqu'au retour au rythme sinusal.

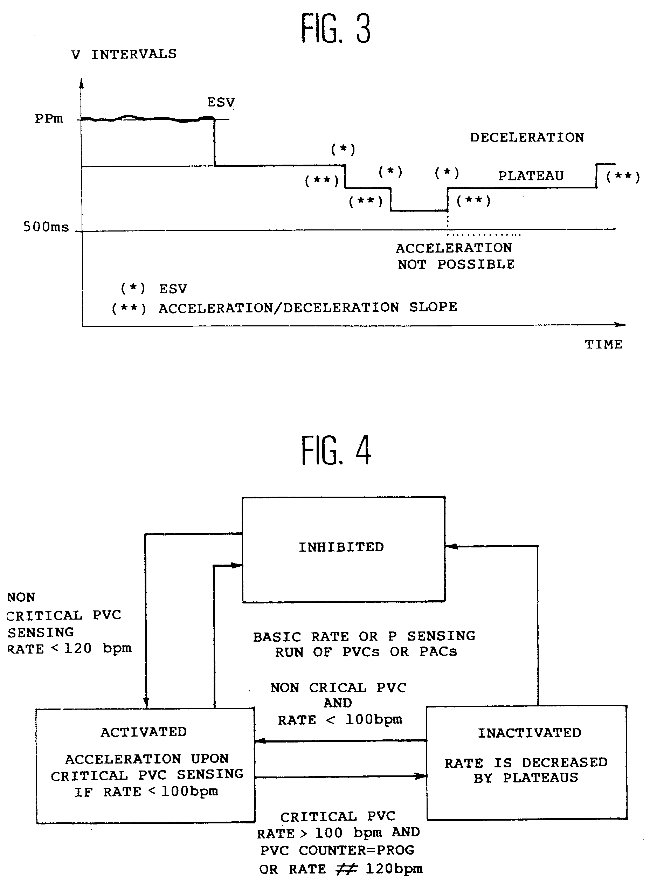

La figure 3 représente le cas d'une succession d'ESV. Lors de la détection de la première ESV, non fréquente, l'intervalle ventriculaire est réduit. Avant la fin du palier, la deuxième ESV, fréquente, est détectée (l'intervalle est de nouveau réduit), puis la troisième ESV, fréquente, est détectée (l'intervalle est encore réduit). Lors de la détection de la quatrième ESV, fréquente l'augmentation de la fréquence n'est plus possible car elle conduirait à un intervalle d'échappement ventriculaire inférieur à 500 ms (fréquence supérieure à 120 cpm).

L'algorithme est alors désactivé, et la fréquence ventriculaire est ralentie par paliers (allongement de l'intervalle d'une pente de décélération) comme dans le cas de la figure 2.

La figure 4 reprend les différentes opérations décrites ci-dessus.

Le procédé de commande du stimulateur cardiaque selon l'invention met en jeu un algorithme. L'entrée dans cet algorithme est déclenchée par la détection d'une ESV à la condition que le rythme cardiaque moyen ne soit pas trop rapide, c'est-à-dire que dans une proportion de préférence égale à 87,5%, l'intervalle moyen entre deux ondes P successives, PPm, soit supérieur à une durée déterminée, par exemple 500 ms. L'intervalle PPm est défini comme l'intervalle moyen, de préférence sur 8 cycles cardiaques, entre deux ondes P consécutives situées en dehors de la période réfractaire auriculaire post-auriculaire ou PRAPA qui est déclenchée par une onde P. Les ondes P hors PRAPA déclenchent un délai auriculo-ventriculaire ou DAV.

L'activation de l'algorithme se traduit par trois sortes d'opérations :

- Tout d'abord le déclenchement de plusieurs périodes (Fig. 1) :

    . une période réfractaire absolue ventriculaire PRAV ,

    . une période réfractaire absolue auriculaire PRAA, plus courte que la PRAV et de préférence égale à (PRAV-94 ms) ,

    . un intervalle d'échappement ventriculaire IEV égal à un pourcentage X de l'intervalle PPm, compris entre 50 et 100 et de préférence égal à 87,5% ,

    . un délai ventriculo-auriculaire VA plus court que l'IEV et de préférence égal à (IEV - 31 ms),

- Ensuite une stimulation de l'oreillette, si la période de limitation de la fréquence auriculaire PLFA déclenchée par l'événement auriculaire qui précède est terminée. La PLFA est de préférence égale à 400 ms.

Sur toute détection d'une onde P hors PRAPA, une nouvelle PRAPA est déclenchée, égale soit à 87,5% de l'intervalle PP précédent si cet intervalle PP était supérieur à la PRAPA, soit 87,5% de l'intervalle moyen PPm dans le cas contraire.

- Enfin, l'initialisation d'un compteur de fonctionnement de l'algorithme, "same IEV counter", qui permet de compter le nombre de cycles ventriculaires avec le même intervalle d'échappement IEV. Le compteur est initialisé à un nombre programmé à "same IEV number", par exemple égal à 20, et il procède par décrément jusqu'à 0.

Après détection de l'ESV et entrée dans l'algorithme, le procédé selon l'invention a un triple objectif :

- assurer une commande plus rapide de l'oreillette ou "over-driving", c'est-à-dire stimuler l'oreillette après un délai VA de plus en plus court pendant quelques cycles, alors que l'IEV reste constant ,

- tenter de rétablir la conduction auriculo-ventriculaire en allongeant progressivement le délai auriculo-ventriculaire ou DAV,

- diminuer progressivement la fréquence ventriculaire en allongeant l'IEV chaque fois que le compteur revient à la valeur "same IEV number", jusqu'à retrouver une détection sinusale (hors PRAPA) ou atteindre la fréquence de base programmée. Cette fréquence de base correspond par exemple à 60 cpm.

Le fonctionnement de l'algorithme s'analyse de la manière suivante en fonction d un événement auriculaire (détection ou stimulation) :

- En l'absence de détection auriculaire durant l'intervalle VA, l'oreillette est stimulée à la fin de cet intervalle et un délai auriculo-ventriculaire est déclenché, de durée : DAV = (IEV - VA) .

- En cas de détection auriculaire durant l'intervalle VA et en dehors de la PRAPA, le rythme sinusal est retrouvé et en conséquence, il y a inhibition de l'algorithme.

- En cas de détection auriculaire dans la PRAPA, cette détection correspond à une extra-systole auriculaire ou ESA, et un nouveau délai VA est déclenché, avec une durée qui correspond au maximum des trois délais suivants : PRAPA, PLFA, et (IEV - 31ms).

En fonction d'un événement ventriculaire, le fonctionnement de l'algorithme s'analyse de la manière suivante.

- En l'absence de détection ventriculaire pendant l'IEV, le ventricule est stimulé à la fin de l'IEV. Afin de retrouver une conduction auriculo-ventriculaire spontanée, la fréquence auriculaire est augmentée par incréments et le délai AV est augmenté corrélativement pour conserver le même IEV pendant le nombre de cycles programmé pour le computeur "same IEV counter". Ensuite, et à chaque fois que le compteur est à la valeur programmée "same IEV number", l'IEV est augmenté par incréments.

Sur la stimulation ventriculaire en fin d'IEV et tant que le nombre programmé de cycles avec le même IEV n'est pas atteint (20 par exemple), le compteur "same IEV counter" est décrémenté, et le délai VA est diminué d'une pente "AV delay increment". De ce fait, à chaque cycle, le DAV est augmenté corrélativement, tant qu'il n'a pas retrouvé sa valeur programmée.

Lorsque le nombre programmé de cycles avec le même IEV est atteint, le compteur est à 0. L'algorithme déclenche alors :

. la ré-initialisation du compteur à la valeur programmée "same IEV number",

. l'augmentation de l'IEV d'une pente "IEV incrément",

. la modification du délai VA de façon à augmenter le DAV d'un incrément "AV delay increment", c'est-à-dire que le délai VA devient égal à :

VA + "IEV increment" - "AV delay increment".

Le DAV est augmenté tant que la conduction auriculo-ventriculaire spontanée n'est pas retrouvée, et tant que le DAV n'a pas atteint sa valeur programmée.

- En cas de détection ventriculaire synchrone durant l'IEV, la conduction auriculo-ventriculaire spontanée est retrouvée. Il n'est plus nécessaire d'augmenter le DAV, mais il faut continuer à augmenter progressivement l'IEV pour atteindre la période de base ou pour retrouver une détection sinusale.

Sur la détection ventriculaire, et tant que le nombre programmé de cycles avec le même IEV n'est pas atteint (20 par exemple), le compteur "same IEV counter" est décrémenté, et les délais VA et IEV sont maintenus constants.

Lorsque le nombre programmé de cycles avec le même IEV est atteint, le compteur est à 0. L'algorithme déclenche alors :

. la ré-initialisation du compteur à la valeur programmée "same IEV number" ,

. l'augmentation de l'IEV de la pente "IEV increment", de 10 à 200 ms et de préférence égale à 60 ms,

. la modification du délai VA de façon à ne pas modifier le DAV, c'est-à-dire que le délai VA devient égal à (VA + "IEV increment").

L'IEV reste constant pendant le nombre programmé de cycles, 20 par exemple, et l'algorithme déclenche une phase identique à la précédente : ré-initialisation du compteur, augmentation de l'IEV, modification du délai VA. Cette phase se reproduit jusqu'à ce que l'IEV devienne égal à la période de base du stimulateur cardiaque. Elle est interrompue en cas de détection auriculaire hors PRAPA, c'est-à-dire lorsque le rythme sinusal est retrouvé.

- En cas de détection d'une nouvelle ESV, il faut distinguer trois cas suivant que cette nouvelle ESV est, ou non, consécutive à une ESV, ou qu'elle est non fréquente.

Si la nouvelle ESV est non fréquente, l'algorithme est activé, ce qui se traduit par les opérations suivantes :

. Déclenchement d'une période réfractaire absolue ventriculaire PRAV,

. Déclenchement d'une période réfractaire absolue auriculaire PRAA plus courte que la PRAV et de préférence égale à (PRAV-94ms),

. Initialisation du compteur de fonctionnement de l'algorithme "same IEV counter".

Si la nouvelle ESV est fréquente, mais n'est pas consécutive à une ESV, l'algorithme s'applique à nouveau, si l'IEV est supérieur à (500 ms + "IEV increment"), en augmentant la fréquence ventriculaire et en conservant le DAV. L'algorithme déclenche alors :

. la ré-initialisation du compteur à la valeur programmée "same IEV number" ,

. la diminution de l'IEV de la valeur "IEV increment" correspondant à la pente d'accélération,

. l'application d'un délai VA égal à (IEV-DAV).

Si l'IEV est inférieur à (500 ms + "IEV increment"), la situation s'analyse comme un échec de l'algorithme en matière de prévention des ESV. Il en résulte une désactivation de l'algorithme (Figure 4).

Si la nouvelle ESV est consécutive à une ESV, la diminution de l'IEV n'est pas appliquée car elle risquerait de déclencher une tachycardie ventriculaire par stimulation prématurée d'une salve d'ESV. Un doublet d'ESV commande donc une inhibition de l'algorithme, tant que la salve d'ESV n'est pas terminée.

L'inhibition de l'algorithme intervient dans les cas suivants :

- sur une détection auriculaire hors PRAPA, car le rythme sinusal est retrouvé ;

- sur deux extra-systoles auriculaires consécutives dans une même PRAPA, car un trouble du rythme auriculaire a pu s'établir et il faut passer en procédure de repli ;

- sur une détection ventriculaire ou une stimulation ventriculaire lorsque l'IEV a atteint la période de base (qui correspond à la fréquence de base, et qui est de 1000 ms dans l'exemple décrit où la fréquence de base est de 60 cpm) ;

- sur deux ESV consécutives, qui peuvent correspondre au déclenchement d'une salve d'ESV, avec inhibition de l'algorithme jusqu'à ce qu'intervienne un événement ventriculaire non-extra-systolique.

En cas d'inhibition de l'algorithme, l'IEV est forcé à la valeur de l'IEV lissé, et le DAV est forcé à la valeur du DAV programmé.

Selon l'invention, un compteur d'ESV est prévu, dont la valeur peut être comprise entre 0 et une valeur programmée, par exemple 50. Ce compteur d'ESV est remis à zéro lors du passage de l'algorithme à son état activé. Ce compteur d'ESV est incrémenté à chaque détection d'une ESV fréquente, et décrémenté à chaque détection d'une ESV non fréquente. La caractérisation des ESV se poursuit de façon continue quel que soit l'état dans lequel est l'algorithme.

Le comptage des ESV n'a lieu que dans l'état activé de l'algorithme. Lorsque le compteur d'ESV atteint sa valeur programmée (par exemple 50), la situation s'analyse comme un échec de la thérapie.

Lorsque la fréquence de stimulation cardiaque dépasse la fréquence maximale d'accélération, par exemple 100 cpm, et que le compteur d'ESV atteint sa valeur programmée, alors l'algorithme est désactivé (Fig. 4) à la détection de la première ESV fréquente.

Ainsi l'algorithme est désactivé sur détection d'une ESV fréquente :

- lorsque l'IEV ne peut être réduit, après détection d'une ESV, sans tomber au dessous de sa valeur limite, 500 ms dans l'exemple décrit, ou

- lorsque la fréquence de stimulation cardiaque est supérieure à la fréquence maximale d'accélération (100 cpm) et que le compteur d'ESV atteint sa valeur programmée (50).

L'algorithme est de nouveau activé lorsque :

- la fréquence de stimulation est inférieure à la fréquence maximale d'accélération (100 cpm),et

- une nouvelle ESV non fréquente est détectée.

## Revendications

1. Stimulateur cardiaque dans lequel en cas de détection d'une d'extra-systole ventriculaire ESV, une stimulation synchrone de l'oreillette est déclenchée , caractérisé en ce que, sur la détection de l'extrasystole, un algorithme est mis en jeu pour déclencher en outre : une commande de la stimulation de l'oreillette à un rythme plus rapide que celui du ventricule pendant quelques cycles cardiaques, une commande de la stimulation synchrone du ventricule pendant une période multiple d'un nombre programmé de cycles cardiaques, et après ce nombre programmé de cycles cardiaques, une diminution du rythme de la commande de la stimulation du ventricule, jusqu'à atteindre la fréquence de base programmée ou retrouver une détection sinusale.

2. Stimulateur selon la revendication 1 caractérisé en ce que l'ESV sert de point de départ à un intervalle d'échappement ventriculaire IEV et a un intervalle ventriculo-auriculaire VA plus court que l'IEV.

3. Stimulateur selon la revendication 2 caractérisé en ce que, l'IEV est égal a un pourcentage de l'intervalle moyen entre deux ondes P successives, compris entre 50 et 100 et de préférence égal a 87,5%.

4. Stimulateur selon la revendication 2 caractérisé en ce que, pendant le nombre programmé de cycles, de préférence égal a 20, l'intervalle d'échappement ventriculaire IEV est maintenu constant, et l'intervalle ventriculo-auriculaire VA est réduit, à chaque cycle, tandis que le délai auriculo-ventriculaire DAV est augmenté de la durée correspondante pour respecter l'égalité : IEV = VA + DAV .

5. Stimulateur selon la revendication 4 caractérisé en ce que, après le nombre programmé de cycles, de préférence égal à 20, l'IEV est augmenté et reste constant pour ledit nombre programmé de cycles.

6. Stimulateur selon la revendication 1 caractérisé en ce que, le nombre programmé de cycles est compté par un compteur initialisé par la détection de l'ESV.

7. Stimulateur selon la revendication 1 caractérisé en ce que, l'algorithme n'est mis en jeu que si un pourcentage de l'intervalle moyen PPm entre deux ondes P successives est supérieur à une durée prédéterminée, de préférence égale à 500 ms.

8. Stimulateur selon la revendication 4, caractérisé en ce qu'en cas de détection d'une nouvelle ESV fréquente, le rythme ventriculaire est de nouveau augmenté d'une pente d'accélération.

9. Stimulateur selon la revendication 8, caractérisé en ce que si le rythme ventriculaire accéléré correspond à un intervalle d'échappement ventriculaire inférieur à 500 ms, alors l'algorithme est désactivé.

10. Stimulateur selon la revendication 9, caractérisé en ce qu'après désactivation de l'algorithme, l'intervalle d'échappement ventriculaire est allongé par paliers jusqu'à retrouver la période de base ou un rythme sinusal.

11. Stimulateur selon la revendication 1, caractérisé en ce que l'algorithme est désactivé sur détection d'une ESV fréquente lorsque l'IEV ne peut être réduit sans tomber au dessous de sa valeur limite égale de préférence a 500ms, ou bien lorsque la fréquence de stimulation cardiaque est supérieure à la fréquence maximale d'accélération, égale de préférence à 100 cpm, et que le compteur d'ESV atteint sa valeur programmée.

12. Stimulateur selon la revendication 11, caractérisé en ce que l'algorithme est de nouveau activé lorsque la fréquence de stimulation est inférieure a la fréquence maximale d'accélération, égale de préférence a 100 cpm, et qu'une nouvelle ESV non fréquente est détectée.

**Claims**

1. A cardiac pacemaker in which synchronous pacing of the atrium is triggered in the event of the detection of a ventricular extrasystole ESV, characterised in that, upon the detection of the extrasystole, an algorithm is brought into play to trigger furthermore: control of the pacing of the atrium at a faster rate than that of the ventricle for several cardiac cycles, control of the synchronous pacing of the ventricle for a period which is a multiple of a programmed number of cardiac cycles, and after this programmed number of cardiac cycles a decrease in the rate of the control of the pacing of the ventricle until the programmed base rate is reached or a sinusal detection is encountered.

2. A pacemaker according to Claim 1, characterised in that the ESV serves as the starting point for a ventricular escape interval IEV and for an atrioventricular interval VA which is shorter than the IEV.

3. A pacemaker according to Claim 2, characterised in that the IEV is equal to a percentage of the average interval between two successive P waves, of between 50 and 100 and preferably 87.5%.

4. A pacemaker according to Claim 2, characterised in that, during the programmed number of cycles, preferably equal to 20, the ventricular escape interval IEV is kept constant, and the atrioventricular interval VA is reduced, upon each cycle, whilst the atrioventricular delay DAV is increased by the corresponding duration to maintain the equality of IEV = VA + DAV .

5. A pacemaker according to Claim 4, characterised in that, after the programmed number of cycles, preferably equal to 20, the IEV is increased and remains constant for said programmed number of cycles.

6. A pacemaker according to Claim 1, characterised in that the programmed number of cycles is counted by a counter initialised by the detection of the ESV.

7. A pacemaker according to Claim 1, characterised in that the algorithm is only brought into play if a percentage of the average interval PPm between two successive P waves is greater than a predetermined duration, preferably equal to 500 ms.

8. A pacemaker according to Claim 4, characterised in that in the event of the detection of a new frequent ESV the ventricular rhythm is again increased by an acceleration slope.

9. A pacemaker according to Claim 8, characterised in that if the accelerated ventricular rhythm corresponds to a ventricular escape interval less than 500 ms, then the algorithm is deactivated.

10. A pacemaker according to Claim 9, characterised in that after deactivation of the algorithm the ventricular escape interval is extended by stages until the base period or a sinus rhythm is encountered.

11. A pacemaker according to Claim 1, characterised in that the algorithm is deactivated upon detection of a frequent ESV when the IEV cannot be reduced without dropping below its limit value preferably equal to 500 ms, or alternatively when the cardiac pacing rate is greater than the maximum acceleration rate, preferably equal to 100 beats per minute, and the ESV counter reaches its programmed value.

12. A pacemaker according to Claim 11, characterised in that the algorithm is activated once again when the pacing rate is less than the maximum acceleration rate, preferably equal to 100 beats per minute, and a new non-frequent ESV is detected.

**Patentansprüche**

1. Herzschrittmacher, bei dem im Fall der Erfassung einer ventrikulären Extra-Systole ESV eine synchrone Stimulation des Herzohrs ausgelöst wird, **dadurch gekennzeichnet, daß** auf die Erfassung der Extra-Systole hin ein Algorithmus in Gang gesetzt wird, um unter anderem auszulösen:

> eine Ansteuerung zur Stimulation des Herzohrs mit einem Rhythmus, der schneller ist als der des Ventrikels während einiger Herzzyklen, eine Ansteuerung zur synchronen Stimulation des Ventrikels während einer Phase, die ein Vielfaches einer programmierten Anzahl von Herzzyklen beträgt, und, nach der programmierten Anzahl von Herzzyklen, eine Verringerung des Rhythmus der Ansteuerung des Ventrikels bis zum Erreichen der programmierten Grundfrequenz oder dem Wiederauffinden einer Sinus-Erfassung.

2. Herzschrittmacher nach Anspruch 1, **dadurch gekennzeichnet, daß** die ESV von dem Ausgangspunkt aus als ein ventrikuläres Ausströmintervall IEV und als ein ventrikulär-aurikuläres Intervall VA dient, das sehr viel kürzer als das IEV ist.

3. Herzschrittmacher nach Anspruch 2,

**dadurch gekennzeichnet, daß** das IEV gleich einem prozentualen Anteil des mittleren Intervalls zwischen zwei aufeinanderfolgenden P-Zacken ist, der zwischen 50% und 100% liegt und vorzugsweise gleich 87,5% ist.

4. Herzschrittmacher nach Anspruch 2, **dadurch gekennzeichnet, daß** während der programmierten Anzahl von Zyklen, vorzugsweise 20, das ventrikuläre Ausströmintervall IEV konstant beibehalten wird und das ventrikulär-aurikuläre Intervall VA bei jedem Zyklus verringert wird, während die aurikulär-ventrikuläre Verzögerung DAV um die entsprechende Dauer erhöht wird, um die Gleichheit zu berücksichtigen: IEV = VA + DAV .

5. Herzschrittmacher nach Anspruch 4, **dadurch gekennzeichnet, daß** nach der programmierten Anzahl von Zyklen, vorzugsweise 20, das IEV erhöht wird und für diese programmierte Anzahl von Zyklen konstant bleibt.

6. Herzschrittmacher nach Anspruch 1, **dadurch gekennzeichnet, daß** die programmierte Anzahl von Zyklen durch einen Zähler gezählt wird, der durch die Erfassung der ESV initialisiert wird.

7. Herzschrittmacher nach Anspruch 1, **dadurch gekennzeichnet, daß** der Algorithmus nur ausgelöst wird, wenn ein prozentualer Anteil des mittleren Intervalls PPm zwischen zwei aufeinanderfolgenden P-Zacken größer als eine vorbestimmte Dauer von vorzugsweise 500 ms ist.

8. Herzschrittmacher nach Anspruch 4, **dadurch gekennzeichnet, daß** im Fall der Erfassung einer neuen, häufigen ESV der ventrikuläre Rhythmus erneut um eine Beschleunigungsstufe erhöht wird.

9. Herzschrittmacher nach Anspruch 8, **dadurch gekennzeichnet, daß** der Algorithmus deaktiviert wird, wenn der beschleunigte ventrikuläre Rhythmus einem ventrikulären Ausströmintervall von weniger als 500 ms entspricht.

10. Herzschrittmacher nach Anspruch 9, **dadurch gekennzeichnet, daß** nach der Deaktivierung des Algorithmus das ventrikuläre Ausströmintervall stufenweise bis zum Wiederauffinden der Grundphase oder eines Sinusrhythmus ausgedehnt wird.

11. Herzschrittmacher nach Anspruch 1, **dadurch gekennzeichnet, daß**

der Algorithmus auf die Erfassung einer häufigen ESV hin deaktiviert wird, wenn das IEV nicht verringert werden kann, ohne unter seinen Grenzwert von vorzugsweise 500 ms abzufallen, oder aber, wenn die Frequenz der Herzstimulation größer als die Maximalbeschleunigungsfrequenz von vorzugsweise 100/min ist und der Zähler der ESV seinen programmierten Wert erreicht.

12. Herzschrittmacher nach Anspruch 11, **dadurch gekennzeichnet, daß** der Algorithmus erneut aktiviert wird, wenn die Stimulationsfrequenz kleiner als eine maximale Beschleunigungsfrequenz von vorzugsweise 100/min ist und eine neue, nicht häufige ESV erfaßt wird.

# FIG. 1

P    R    ESV

stim  A

> 300 ms

PRAV

PRAA

IEV=X% PPm

VA=IEV-31ms

# FIG. 2

V INTERVALS

PPm                    ESV              SINUS RHYTHM

PLATEAU

87,5 % OF PPm        PLATEAU        RE-ESTABLISHED

IEV INCREMENT

TIME

# FIG. 3

V INTERVALS

PPm

ESV

DECELERATION

(*)

(*)     (*)     PLATEAU     (**)

(**)

(**)

(**)     (**)

500ms

ACCELERATION
NOT POSSIBLE

(*) ESV
(**) ACCELERATION/DECELERATION SLOPE

TIME

# FIG. 4

INHIBITED

NON
CRITICAL PVC
SENSING
RATE < 120 bpm

BASIC RATE OR P SENSING
RUN OF PVCs OR PACs

NON CRICAL PVC
AND
RATE < 100bpm

ACTIVATED

ACCELERATION UPON
CRITICAL PVC SENSING
IF RATE < 100bpm

INACTIVATED

RATE IS DECREASED
BY PLATEAUS

CRITICAL PVC
RATE > 100 bpm AND
PVC COUNTER=PROG
OR RATE ≠ 120bpm